Europäisches Patentamt

European Patent Office

Office européen des brevets

(11) Publication number: **0 159 749**
**A1**

## (12) EUROPEAN PATENT APPLICATION

(21) Application number: **85200511.5**

(22) Date of filing: **02.04.85**

(51) Int. Cl.⁴: **A 61 K 39/29**

(30) Priority: **04.04.84 NL 8401066**

(43) Date of publication of application: **30.10.85**
**Bulletin 85/44**

(84) Designated Contracting States: **AT BE CH DE FR GB IT LI LU NL SE**

(71) Applicant: **Stichting Centraal Laboratorium van de Bloedtransfusiedienst van het Nederlandse Rode Kruis, Plesmanlaan 125, NL-1066 CX Amsterdam (NL)**

(72) Inventor: **Reesink, Hendrik Willem, Plesmanlaan 125, NL-1066 CX Amsterdam (NL)**
Inventor: **Lelie, Peter Nicolaas, Plesmanlaan 125, NL-1066 CX Amsterdam (NL)**
Inventor: **Visser, Biense Thomas, Plesmanlaan 125, NL-1066 CX Amsterdam (NL)**

(74) Representative: **Kooy, Leendert Willem et al, OCTROOIBUREAU VRIESENDORP & GAADE P.O. Box 266, NL-2501 AW The Hague (NL)**

(54) Activated hepatitis B surface antigen product.

㊗ A vaccine containing an activated HBs antigen product, originally isolated from human HBsAg containing plasma, prepared synthetically, or by DNA recombinant techniques and containing 0.1–99.5 % of other proteins from human and/or animal origin based on the total amount of protein material in the vaccine, not being added anti-HBs antibodies, in which the HBs Ag particles are aggregated to agglomerates having a thickness of 18–22 nanometers and a length of 36 nanometers at a minimum or in which vaccine the single HBsAg particles are complexed with plasma proteins of human or animal origin.

ACTIVATED HBsAg PRODUCT

Vazcines against hepatitis are known for instance
from British patent specification 1 282 984.

The invention provides a vaccine contains
hepatitis B surface-antigen (HBsAg) isolated from
human HBsAg containing blood plasma, prepared
synthetically or by DNA-recombinant techniques which
vaccine is 10-100 times more immunogenic than the
commercially available hepatitis B vaccines.

A vaccine according to the invention contains an
activated HBs antigen product as mentioned above and
0.1 - 99.5% human and/or animal protein material based
on its total protein content not being anti-HBs
antibodies, in which the HBsAg particles are
aggregated to agglomerates having a thickness of
18-22 nanometers and a length of 36 nanometers at a
minimum or in which vaccine the single HBsAg particles
are complexed with plasma proteins of human or animal
origin.

Such a vaccine can be prepared by mixing human HBsAg
positive blood plasma with an aqueous polyethylene
glycol solution (PEG) to a final concentration of 5%
(weight/volume). After removal of the precipitate containing the bulk of infectious Dane-particles, the HBsAg containing supernatant is subjected to purification steps in which HBsAg is partly purified. Suitable purification methods are:

1. precipitation with 6% PEG at pH 3.5, followed by ultracentrifugation of the resuspendate at 200,000 x g for $2\frac{1}{2}$ - $3\frac{1}{2}$ hours.

2. Immuneadsorption on a human, animal or monoclonal anti-HBs conjugated column, desorption with 3 molar sodium thiocyanate (NaCNS) and dialysis against Phosphate Buffer Saline (PBS).

Such HBsAg preparations contain single 18-22 nm particles, which are about 10 times more immunogenic in aqueous solution in mice potency tests than highly purified HBsAg particles, probably because of the presence of plasma proteins complexed to HBsAg, playing an important role in contracting immunity in vivo.

Such an HBsAg protein mixture containing 0.1-99.5% HBsAg based on the total amount of proteins is subjected to heat treatment in the range of 60-400$^{\circ}$C, preferably in the range of 80-120$^{\circ}$C.
Generally the heat treatment is carried out at a temperature in the range of 85.110$^{\circ}$C.

In consequence of this heating procedure the single HBsAg particles are aggregated to agglomerates and the immunogenicity is significantly increased.

A vaccine containing such an activated HBs antigen product according to the invention may contain only 0.1 - 10% by weight of HBsAg proteins based on the total amount of proteins.

However, it is also possible to subject a more purified HBsAg product to the heat treatment mentioned above, provided that purification methods leading to highly purified HBsAg particles and removing of associated proteins from the surface antigen, such as equilibrium centrifugation in CsCl or KBr gradients, are avoided.

### Example 1

Human blood plasma which contained Hepatitis B surface antigen (HBsAg) was mixed with Freon 113 (Du Pont de Nemours Int. S.A., Switzerland) in a volume ratio of 2 : 1 at room temperature for half an hour. After centrifugation three layers were formed consisting of a solution of extracted lipoid material, a layer of clear plasma and thereinbetween a precipitate. 10 Parts of the clear plasma protein solution (=Vo) were mixed with 25 parts of an 0.15 molar phosphate buffered saline (PBS) solution (pH 7.4) and 5 parts of a 40% by weight's aqueous polyethylene glycol solution (PEG 6000, final concentration 5% weights bu volume). The precipitate formed after an incubation of 1 night at 4°C was centrifuged off and removed and to the supernatant a 40% by weight's aqueous PEG 6000 solution was added to a final concentration of 6% (weight/volume).

By addition of a 2.5 molar hydrochloric acid solution the pH thereof was adjusted to 3.5. After an incubation during 1 night the precipitate formed was centrifuged off and the supernatant was removed. The precipitate was suspended in 1/5 of the original volume (=Vo) by addition of a PBS solution (pH 7.4). By addition of a 2.5 molar sodium hydroxyde the pH was adjusted to 7.4. By ultracentrifuging this solution at 200,000 x g for 3.5 hours a sediment was obtained. This sediment which consists of HBsAg proteins only for a small portion and consists of human plasma proteins for the remainder, was suspended by addition of 1/10 of the original volume (=Vo) phosphate buffered saline (PBS) solution (pH 7.4).

For 90 seconds this protein mixture was heated to 101.5 - 104 °C in a closed continuous flow system. Because of the overpressure in the system, the solution did not boil. Under these circumstances the non-heat-resistant plasma proteins precipitated, but the major part of the HBsAg protein remained in solution.

After removal of the insoluble proteins by ultracentrifuging for 1 hour at 48,000 x g and addition of Tween 80 to a final concentration of 50 mg/l the aqueous protein solution of the vaccine, consisting for at least of 0.5% by weight of specific HBsAg proteins, was filtered in a sterile way and collected in a presterilized suspension of aluminium phosphate in PBS (pH=7.4). The final suspension contained 3 µg HBsAg and 25 millimol aluminium phosphate per ml.

Glass ampoules were filled with doses of 1 ml. The ampoule was heated to 65°C for a further 10 hours (the so-called pasteurisation).

When comparing a vaccine, as described above, with currently available HB-vaccines consisting of highly purified HBsAg particles and which were not heated at 101.5 - 104°C, it appeared that when tested in mice and humans the above vaccine was 10-100 times more immunogenic.

In accordance therewith it turned out that in humans with a normal immune reactivity the above vaccine could be administered in a 10-100 times lower specific antigen dose than HB-vaccines which were not prepared according to the invention.

Example 2

10 Parts of human plasma which contained hepatitis B surface antigen (HBsAg) were mixed with 25 parts of 0.15 molar phosphate buffer Saline (PBS) solution (pH 7.4) and 5 parts of a 40% by weight's aqueous polyethylene glycol solution (PEG 6000, final concentration 5% weight/volume). The precipitate formed after incubation overnight at 4°C was centrifuged off and removed. The obtained 5% PEG supernatant was incubated for 2-16 hours with a column conjugated with human polyclonal or monoclonal anti-HBs. Per international unit anti-HBs conjugated to the sepharose 100-1000 nanograms of HBsAg in the 5% PEG supernatant were incubated with the column. Not specifically bound proteins were washed away from the column with PBS (4-6 times column volume). The specifically bound HBsAg-particles and associated plasma proteins are eluted from the column with 2-4 times column volume of a 3 molar sodium thiocyanate (NaSCN) solution in PBS (pH 7.4) followed by 2-4 times column volume PBS.

The HBsAg-protein mixture in the NaCNS-eluate is concentrated and the NaCNS is removed by dialysis against PBS.

To the thus formed HBsAg concentrate, containing also plasma proteins (predominantly human albumin and immunoglobulins) in a range of 0.5 to 10% of the total protein content in the preparation, was added Tween-80 to a final concentration of 50 mg/l.

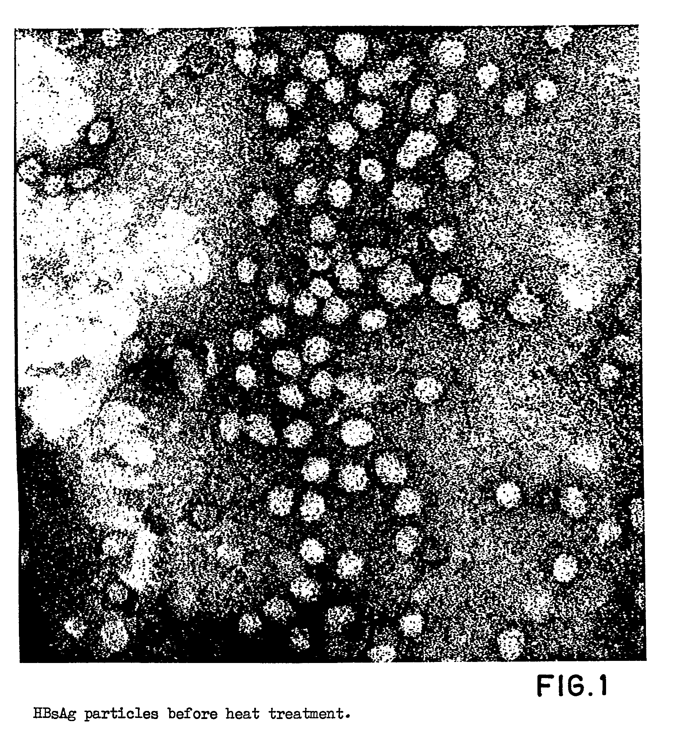

Figure 1 shows an electron microscopy picture of a thus obtained preparation in which single 18-22 nm HBsAg particles can be seen.

For 90 seconds this protein mixture containing 40-400 μg HBsAg was heated to 101.5-104°C in a closed system. Because of the overpressure in the system the solution did not boil. Under these circumstances the non-heat resistant plasma proteins, including possible residual heat inactivated Dane-particles, precipitated but the major part of the HBsAg proteins remained in solution. After removal of the insoluble material by centrifugation for 1 hour at 48,000 x g the clarified aqueous solution of the vaccine was filtered in a sterile way through a membrane filter.

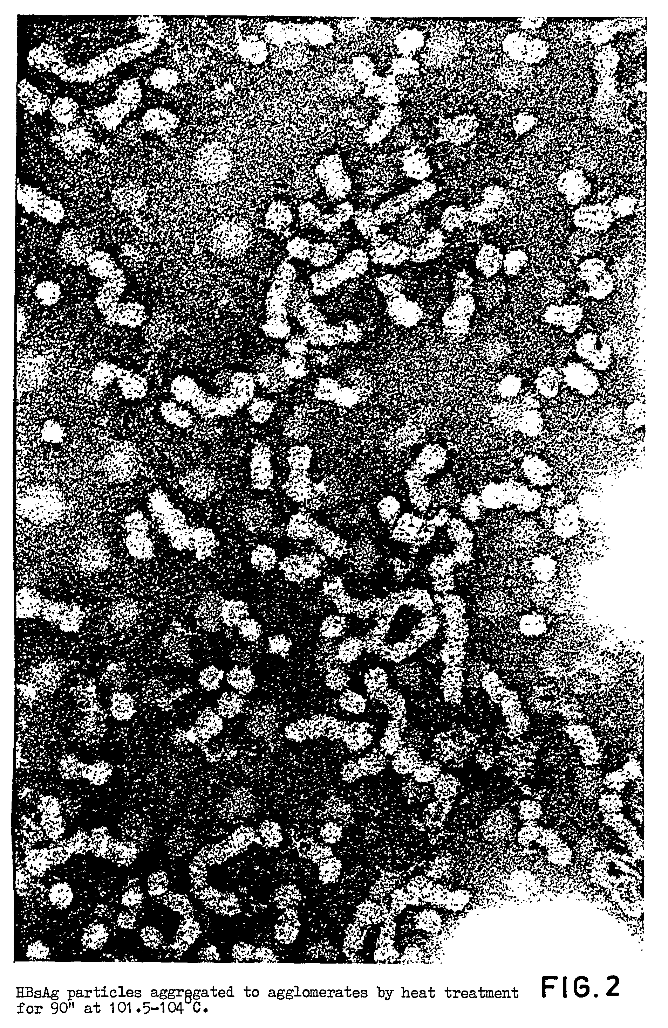

Figure 2 shows an electron microscopy picture of a thus obtained preparation after heat treatment, in

-7-

which longitudinal aggregates of HBsAg particles can be seen.

Finally the aqueous vaccine solution is pasteurized for 10 hours at 65°C either before or after coupling the vaccine to 25 millimolar aluminium phosphate.

When comparing the aqueous solution of the vaccine obtained according to example 2 it appeared in mice potency tests that the vaccine after heat treatment was a factor 10-100 times more immunogenic than an aqueous solution of highly purified HBsAg in which the remaining plasma proteins (albumin and immunoglobulins) were removed by CsCl-gradient centrifugation at 200,000 x g for 40 hours.

0159749

## CLAIMS

1. A vaccine containing an activated HBs antigen product, originally isolated from human HBsAg containing plasma, prepared synthetically, or by DNA recombinant techniques and containing 0.1-99.5% of other proteins from human and/or animal origin based on the total amount of protein material in the vaccine, not being added anti-HBs antibodies, in which the HBsAg particles are aggregated to agglomerates having a thickness of 18-22 nanometers and a length of 36 nanometers at a minimum or in which vaccine the single HBsAg particles are complexed with plasma proteins of human or animal origin.

2. A process of preparing a vaccine according to claim 1, characterized in that human blood plasma which contains Hepatitis B surface antigen (HBsAg) is mixed with an aqueous polyethylene glycol solution to a concentration of polyethylene glycol of 5% (weight/volume), and, after removal of the formed precipitate, the HBsAg, present in the supernatant, is partly to a heat treatment at a temperature in the range of 60-400$^{\circ}$C and to which Tween 80 is added to a final concentration of 50 mg/l.

3. A process according to claim 2, characterized in that the HBsAg product subjected to the heat treatment contains 0.1 - 10% HBsAg based on the total amount of protein in the vaccine.

4. A process according to claim 2 or 3, characterized in that the heat treatment is carried out at a temperature in the range of 80-120°C.

5. A process according to claim 2, 3 or 4, characterized in that the heat treatment is carried out at a temperature in the range of 95-110°C.

6. A process according to claim 2, 3, 4 or 5, characterized in that the HBsAg containing supernatant which is formed after mixing HBsAg plasma with a 5% (weight/volume) polyethylene glycol solution is further purified by immune adsorption of the HBsAg particles to a human anti-HBs, animal anti-HBs or monoclonal anti-HBs conjugated column and desorption of the HBsAg particles with 3 molar sodium thiocyanate (NaCNS).

**FIG.1**

HBsAg particles before heat treatment.

HBsAg particles aggregated to agglomerates by heat treatment
for 90" at 101.5–104°C.                                          FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 100 050 (ALPHA THERAPEUTIC CORP.) * Claim; page 2, lines 15-19 * | 1 | A 61 K 39/29 |
| Y | | 2-6 | |
| Y | EP-A-0 094 611 (CEDARS-SINAI MEDICAL CENTER) * Claims 1,2,9-15,17,28-34,45,50,60-62; page 3, lines 11-20 * | 2-5 | |
| Y | FR-A-2 256 247 (THE GREEN CROSS CORP.) * Claims 1,5 * | 2-5 | |
| Y | US-A-3 735 004 (S. KRUGMAN et al.) * Claims 1-5,10-12 * | 2-5 | TECHNICAL FIELDS SEARCHED (Int. Cl.4) A 61 K |
| Y | GASTROENTEROLOGY, vol. 77, no. 5, 1979, page A46; J.R. WANDS et al. "Production and characterization of monoclonal antibodies to hepatitis B surface antigen (HBsAg) by cellular hybridization" * Page A46, left-hand column, 1st abstract * | 6 | |

The present search report has been drawn up for all claims

| Place of search THE HAGUE | Date of completion of the search 05-07-1985 | Examiner RYCKEBOSCH A.O.A. |
|---|---|---|